# EUROPEAN PATENT SPECIFICATION

(11) **EP 2 121 570 B1**
(45) Date of publication and mention of the grant of the patent: **03.09.2014**
(21) Application number: 07855126.4
(22) Date of filing: 13.12.2007
(51) Int. Cl.: C07C 209/26

(54) **PROCESS FOR THE REDUCTIVE AMINATION OF ALDEHYDES AND KETONES**
VERFAHREN ZUR REDUKTIVEN AMINIERUNG VON ALDEHYDEN UND KETONEN
PROCÉDÉ D'AMINATION RÉDUCTRICE D'ALDÉHYDES ET DE CÉTONES

(30) Priority: 15.12.2006 US 875232 P
(43) Date of publication of application: 25.11.2009
(73) Proprietor: Dow Global Technologies LLC, Midland, MI 48674 (US)
(72) Inventor: MOLITOR, Erich Joseph, Midland, MI 48674 (US); TOYZAN, Todd William, Freeland, MI 48623 (US)
(74) Representative: Beck Greener
(86) International application number: PCT/US2007/087366
(87) International publication number: WO 2008/076795

(56) References cited:
- EP-A- 1 201 642
- EP-A- 1 775 281
- JP-A- 10 130 210
- JP-A- 10 310 559
- JP-A- 11 029 534
- US-A- 5 091 585

## Description

Embodiments disclosed herein relate generally to methods for preparing diamines via a reductive amination process in the presence of methanol.

Bis(aminomethyl)cyclohexane is a diamine that has applications as a precursor to an aliphatic diisocyanate (bis(isocyanatomethyl)cyclohexane). It is useful as a chain extender in certain polyurethanes systems and may be used as an epoxy curing agent. Bis(aminomethyl)cyclohexane exists as a number of isomers, of which the 1,3-and 1,4-isomers are of primary interest. The 1,3- and 1,4-isomers may also exist in a number of diastereomeric forms, as the aminomethyl groups may each reside above or below the plane of the cyclohexane ring.

1,3- and 1,4-bis(aminomethyl)cyclohexane mixtures may be prepared via a number of synthetic routes. A route of interest starts with butadiene and acrolein, which forms 1,2,3,6-tetrahydrobenzaldehyde in a Diels-Alder reaction. This intermediate is then hydroformylated to add a second aldehyde group and reductively aminated to form the desired diamine. A mixture of isomeric forms of the diamine is obtained. *See, e.g.,* U. S. Patent No. 6,252,121.

The reductive amination of hydroformylated 1,2,3,6-tetrahydrobenzaldehyde using a Raney metal catalyst or nickel on silica gel/alumina, as in U. S. Patent No. 6,252,121, tends to produce the desired diamine product in low yields. A significant portion of the starting material forms unwanted by-products and polymeric species. As a result, raw material costs are high and purification of the crude product may be difficult and expensive. Polymeric by-products often foul the reactor.

It is sometimes possible to suppress by product formation in reductive amination reactions by "protecting" (or "blocking") the aldehyde groups with an alkyl amine. See, e. g., U. S. Patent Nos. 5,041,675 and 5,055,618. The blocked groups are more resistant to polymerization and other unwanted side reactions. However, this approach requires the use of additional raw materials and introduces additional chemical species into the reaction, which must later be removed from the crude product and recycled. Process yields are still far short of those that are needed to have a highly economical process.

Process yields of greater than 90 percent have been obtained for some amination reactions. For example, JP 10130210 reports mixing of nonanedial and 2-methyloctanedial with triethylamine in methanol and feeding the mixture to a reactor containing Raney Ni, ammonia, hydrogen, and methanol to produce nonanediamine and 2-methyloctanediamine at a 92 percent yield. Similarly, JP07196586 uses tert-butyl alcohol and a nickel catalyst supported on diatomaceous earth. As another example, EP628535 describes the reductive amination of certain aldehydes to give primary amines in greater than 90 percent yields by reaction with ammonia and hydrogen in methanol using a nickel catalyst.

Accordingly, there exists a need for methods by which cycloaliphatic bis(aminomethyl) compounds may be prepared economically and in high yield.

The present invention provides a method for reductively aminating a starting aldehyde or ketone compound having at least two aldehyde or ketone groups per molecule to form a product amine compound, comprising (a) mixing the starting aldehyde or ketone compound with a quantity of the product amine compound and methanol to form a liquid mixture, and (b) subjecting the liquid mixture to reductive amination conditions in the presence of ammonia and hydrogen to produce additional product amine compound, wherein during steps (a) and (b) the molar ratio of product amine compound to starting aldehyde or ketone compound in the mixture is 1:1 or greater.

In another aspect, the invention provides a method for reductively aminating a starting aldehyde or ketone compound having at least two aldehyde or ketone groups per molecule to form a product amine compound, comprising (a) mixing the product amine compound and starting aldehyde or ketone compound at a molar ratio of at least 1:1 and methanol to form a liquid mixture, and maintaining the liquid mixture under non-reductive amination conditions sufficient to form an intermediate mixture containing reaction intermediates formed from the product amine compound and the starting aldehyde or ketone compound, which reaction intermediates consist predominantly of one or more macrocyclic polyimine compounds; and (b) thereafter subjecting at least one of the macrocyclic polyimine compounds to reductive amination conditions in the presence of ammonia and hydrogen to convert the macrocyclic polyimine compound to the product amine compound.

In another aspect, the invention provides a method for reductively aminating a starting aldehyde or ketone compound having at least two aldehyde or ketone groups per molecule to form a product amine compound, comprising (a) mixing product amine compound and the starting aldehyde or ketone compound at a mo lar ratio of at least 1:1 and methanol to form a liquid mixture, and maintaining the liquid mixture at a temperature of 0 to 50 °C for a period of at least 5 minutes to form an intermediate mixture; (b) thereafter subjecting the intermediate mixture to reductive amination conditions in the presence of ammonia and hydrogen to form the product amine compound.

In another aspect, the invention provides a method for reductively aminating a starting aldehyde or ketone compound having at least two aldehyde or ketone groups per molecule to form a product amine compound, comprising subjecting a liquid mixture containing one or more macrocyclic polyimine compounds and methanol to reductive amination conditions in the presence of ammonia and hydrogen to convert the cyclic polyimine compound(s) to the product amine compound, wherein the macrocyclic polyimine compound(s) predominantly contains species having a molecular weight of 450 to 1500.

In another aspect, to the present invention provides a method for reductively aminating an alicyclic dialdehyde or alicyclic diketone compound in which the carbonyl carbons of the aldehyde or ketone groups are attached directly to an alicyclic ring structure, to form a product alicyclic diamine compound. The method may include (a) mixing product alicyclic diamine compound and the starting alicyclic aldehyde or alicyclic ketone compound at a molar ratio of at least 1:1 and methanol to form a liquid mixture, and maintaining the liquid mixture at a temperature of 0 to 50 °C for a period of at least 5 minutes to form an intermediate mixture; and (b) thereafter subjecting at least one component of the intermediate mixture to reductive amination conditions in the presence of ammonia and hydrogen to form the product alicyclic diamine compound.

In another aspect, the invention provides a continuous or semi-continuous method for reductively aminating a starting aldehyde or ketone compound having at least two aldehyde or ketone groups per molecule to form a product amine compound. The method may include continuously or intermittently feeding the starting aldehyde or ketone compound to a reaction zone which is maintained at reductive amination conditions and contains methanol, product amine compound, ammonia, and hydrogen, wherein the starting aldehyde or ketone compound is fed into the reaction zone at a rate such that the molar ratio of product amine compound to starting aldehyde compound in the reaction zone is maintained at 1:1 or higher.

Other aspects and advantages of the invention will be apparent from the following description and the appended claims.

In one aspect, embodiments disclosed herein relate to a method for reductively aminating a starting aldehyde or ketone compound having at least two aldehyde or ketone groups per molecule to form a product amine compound, comprising (a) mixing the starting aldehyde or ketone compound, methanol , and a quantity of the product amine compound to form a liquid mixture, and (b) subjecting the liquid mixture to reductive amination conditions in the presence of ammonia and hydrogen to produce additional product amine compound, wherein during steps (a) and (b) the molar ratio of product amine compound to starting aldehyde or ketone compound in the mixture is 1:1 or greater.

In other aspects, embodiments disclosed herein relate to a method for reductively aminating a starting aldehyde or ketone compound having at least two aldehyde or ketone groups per molecule to form a product amine compound, comprising: a) mixing product amine compound and the starting aldehyde or ketone compound at a molar ratio of at least 1:1 with methanol to form a liquid mixture, and maintaining the liquid mixture under non-reductive amination conditions sufficient to form an intermediate mixture containing reaction intermediates formed from the product amine compound and the starting aldehyde or ketone compound, which reaction intermediates consist predominantly (*i.e*. greater than 50 weight percent, especially 70-99% by weight) of one or more macrocyclic polyimine compounds; and b) thereafter subjecting the reaction intermediates to reductive amination conditions in the presence of ammonia and hydrogen to convert the macrocyclic polyimine compound to the product amine compound.

In other aspects, embodiments disclosed herein relate to a method for reductively aminating a starting aldehyde or ketone compound having two or more aldehyde or ketone groups to form a product amine compound, comprising: a) mixing product amine compound and the starting aldehyde or ketone compound at a molar ratio of at least 1:1 to form a liquid mixture, and maintaining the liquid mixture at a temperature of 0 to 50°C for a period of at least 5 minutes to form an intermediate mixture; and b) thereafter subjecting the intermediate mixture to reductive amination conditions in the presence of ammonia and hydrogen to form the product amine compound.

In other aspects, embodiments disclosed herein relate to a method for reductively aminating an alicyclic dialdehyde or alicyclic diketone compound in which the carbonyl carbons of the aldehyde or ketone groups are attached directly to an alicyclic ring structure, to form a product alicyclic diamine compound, comprising: (a) mixing product alicyclic diamine compound and the starting alicyclic aldehyde or alicyclic ketone compound at a molar ratio of at least 1:1 to form a liquid mixture, and maintaining the solution at a temperature of 0 to 50 °C for a period of at least 5 minutes to form an intermediate mixture; and (b) thereafter subjecting the intermediate mixture to reductive amination conditions in the presence of ammonia and hydrogen to form the product alicyclic diamine compound.

In other aspects, embodiments disclosed herein relate to a continuous or semi-continuous method for reductively aminating a starting aldehyde or ketone compound having at least two aldehyde or ketone groups per molecule to form a product amine compound, comprising continuously or intermittently feeding the starting aldehyde or ketone compound to a reaction zone which is maintained at reductive amination conditions and contains product amine compound, ammonia and hydrogen, wherein the starting aldehyde or ketone compound is fed into the reaction zone at a rate such that the molar ratio of product amine compound to starting aldehyde compound in the reaction zone is maintained at 1:1' or higher.

This process permits the product amine compound to be produced in very high yields, typically at least 70%, at least 80%, at least 90%, at least 92%, at least 93%, at least 94%, at least 95% or even higher, based on the starting aldehyde or ketone compound. Surprisingly, the mixture of product amine with the starting aldehyde or ketone compound does not polymerize to form a high molecular weight polymer. Instead, it is believed that low molecular weight intermediate species are formed that remain soluble in the reaction mixture and are readily converted to form more of the product amine under reductive amination conditions. In embodiments described below as the two-stage process, it is believed that macrocyclic species mostly having molecular weights of 450 or less to 1500 tend to form, together with some linear reaction products of similar molecular weight. A further advantage of this process is that somewhat high concentrations of reactants may be used. This reduces the requirement for solvents and in that manner reduces the volume of material that must be handled. The smaller process volumes reduce the size and therefore the cost of the equipment that is needed to operate the process. The ability to use somewhat high concentrations of starting materials is considered to be quite surprising, as macrocyclic compounds are usually formed only under high dilution conditions (see, for example, H. An, J. S. Bradshaw, R. M. Izatt, Chem. Rev. 1992, 92, 543-572), while high starting material concentrations usually favor the production of high molecular weight, insoluble polymers that are difficult or impossible to reductively aminate.

The process has high selectivity to the desired primary amine products. In particular, unwanted secondary macrocyclic amine compounds are not formed in significant quantities. Selectivity to desired primary amine products has been disclosed in, for example, U.S. Provisional Patent Application No. 60/685,489, filed June 30, 2005, and PCT Application No. PCT/US2006/025559, filed June 29, 2006.

The methods disclosed herein are applicable to making a variety of amine compounds from the corresponding starting aldehyde or ketone compound. The aldehyde or ketone starting material has two or more aldehyde or ketone groups per molecule. It some embodiments, the aldehyde or ketone may contain 2 aldehyde or ketone groups per molecule; and 2 or 3 groups per molecule in other embodiments. The starting aldehyde or ketone compound for use in a two-stage process as described below may be one which is capable of reacting with the product amine compound to form predominantly macrocyclic polyimine compounds. Macrocyclic polyimine formation is favored when (a) the aldehyde or ketone groups are equivalent and (b) when the aldehyde or ketone compound contains a somewhat rigid and/or bulky structure that constrains the spatial relationship between the aldehyde or ketone groups.

Aldehyde or ketone groups are considered to be equivalent for purposes of the present disclosure if the carbon atoms to which the respective carbonyl carbons are attached, plus the adjacent carbon atoms, are identically substituted (or unsubstituted, as the case may be) in each instance. In the case of dialdehydes and diketones, the molecule may be symmetrical about at least one line of symmetry between the carbonyl carbons.

Examples of rigid and/or bulky structures include cycloaliphatic moieties, which may be monocyclic, bicyclic or polycyclic. The cycloaliphatic moiety may contain at least one aliphatic ring structure that contains from 4 to 8 atoms in a ring (although it may also contain other ring structures as well). The carbonyl carbons of the aldehyde or ketone groups may be attached directly to a carbon atom of the ring structure. The ring structure may contain one or more heteroatoms provided that the ring structure is inert to the conditions of the process. Ring structures may include cyclohexane, cyclopentane, cycloheptane and cyclooctane. Such moieties may be substituted with the aldehyde or ketone groups in the 1,2-, 1,3- or 1,4-positions (or 1,5- positions in the case of cyclooctane).

Specific aldehyde and ketone compounds that are useful in various embodiments include 1,3-cyclopentanedicarboxaldehyde, 1,3- and 1,4-cyclohexanedicarbox-aldehyde, 1,3- and 1,4-cycloheptanedicarboxaldehyde, 1,3-, 1,4-, and 1,5-cyclooctanedicarboxaldehyde, tetrahydro-2H-pyran-3,5-dicarbaldehyde, tetrahydro-2H-pyran-2,5-dicarbaldehyde, 1-methylpiperidine-3,5-dicarbaldehyde, 1-methyl-piperidine-2,5-dicarbaldehyde, tetrahydro-2H-thiopyrane-3,5-dicarbaldehyde, tetrahydro-2H-thiopyran-2,5-dicarbaldehyde, 1,3-diacetylcyclopentane, 1,3- and 1,4-diacetylcyclohexane, 1,3- and 1,4-diacetylcycloheptane, 1,3-, 1,4- and 1,5-diacetylcyclooctane.

The product amine compounds contain primary amino groups at the sites of the aldehyde or ketone groups of the starting material. Corresponding product amine compounds include 1,3-bis(aminomethyl)cyclopentane, 1,3- and 1,4-bis(aminomethyl)cyclohexane, 1,3- and 1,4-bis(aminomethyl)cycloheptane, 1,3-, 1,4-, and 1,5-bis(aminomethyl)cyclooctane, 3,5-bis(aminomethyl)tetrahydro-2H-pyran, 2,5-bis(aminomethyl)tetrahydro-2H-pyran, 3,5-bis(aminomethyl)-1-methylpiperidine 2,5-bis(aminomethyl)-1-methylpiperidine, 3,5-bis(aminomethyl)tetrahydro-2H-thiopyran, 2,5-bis(aminomethyl)tetrahydro-2H-thiopyran, 1,3-bis(1-aminoethyl)cyclopentane, 1,3- and 1,4-bis(1-aminoethyl)cyclohexane, 1,3- and 1,4-bis(1-aminoethyl)cycloheptane, 1,3-, 1,4-, and 1,5-bis(1-aminoethyl)cyclooctane.

The process of the present disclosure may be conducted such that the reductive amination reaction is performed on a reaction mixture that contains product amine and starting aldehyde or ketone compound at a molar ratio of at least 1:1. Under these conditions, the starting aldehyde or ketone compound rapidly forms low molecular weight intermediates which are then reductively aminated to form more of the product amine.

In some embodiments, the mixture of product amine and starting aldehyde or ketone compound is formed under reactive amination conditions. Reductive amination conditions typically include (1) the presence of ammonia and hydrogen, (2) superatmospheric pressures, (3) elevated temperatures and (4) the presence of an active hydrogenation catalyst. Embodiments in which the product amine and starting aldehyde or ketone compound are brought together under reductive amination conditions are sometimes referred to herein by the shorthand term "single-stage" processes.

In other embodiments, product amine and starting aldehyde or ketone compound are mixed together under non-reductive amination conditions. Non-reductive amination conditions are those at which no significant reductive amination of the starting aldehyde or ketone compound (or intermediates) occurs. Non-reductive amination conditions include any set of conditions that lack at least one condition that is necessary for the reductive amination to occur. The missing condition may be, for example, the absence of hydrogen or ammonia, the lack of a hydrogenation catalyst, or the lack of sufficient temperature and/or pressure conditions. Two or more of these conditions may be lacking. Processes in which the product amine and starting aldehyde or ketone compound are brought together under non-reductive amination conditions are sometimes referred to herein by the shorthand "two-stage" processes. In the two-stage process, the first reaction stage may be conducted in the absence of the hydrogenation catalyst, at a temperature lower than that required for the reductive amination reaction to significantly occur, or both.

The single-stage process is conveniently conducted by forming a mixture of the product amine, ammonia and hydrogen, and heating the mixture to a temperature sufficient for the reductive amination reaction to proceed. This mixture is then contacted with starting aldehyde or ketone product, which may occur in the presence of a reaction catalyst as described below. The starting aldehyde or ketone compound is added to the reaction mixture at such a rate that the molar ratio of product amine to starting aldehyde or ketone compound in the reaction mixture remains no higher than 1:1. Under the elevated temperatures generally required for the reductive amination to proceed, the product amine and starting aldehyde or ketone compound generally react very rapidly to form intermediates that then react to form more of the product amine. For this reason, instantaneous concentrations of starting aldehyde or ketone compound in the reaction mixture tend to remain small. Similarly, the molar ratio of product amine to starting aldehyde or ketone compound tends to be far in excess of 1:1 in the single-stage process. In some embodiments, the concentration of starting aldehyde or ketone compound in the reaction mixture of a single-stage process is no higher than 35% by weight of the liquid components of the reaction mixture (i.e., product amine, starting aldehyde or ketone compound, intermediates, ammonia and methanol that may be present). In other embodiments, the concentration of starting aldehyde or ketone compound will be lower than 10% by weight; and no more than 5% by weight in yet other embodiments, due to their rapid conversion of the starting material.

The single-stage process is conducted with the starting aldehyde or ketone compound and product amine compound dissolved in methanol. The solvent is such that the starting materials are soluble in the proportions that are present in the reaction mixture. The solvent is not reactive with those materials, or with ammonia or hydrogen, under the conditions of the process. The solvent should not interfere undesirably with the activity of any catalyst that is used for the reductive amination reaction. The solvent should remain a liquid under the conditions of the reductive amination process. High yields and selectivities may be obtained when methanol is used as the solvent. In some embodiments, the concentration of solvent may be from 1 to 99 weight percent of the reaction mixture. In other embodiments, the concentration of solvent may be from 1 to 50 weight percent of the reaction mixture; and from 5 to 15 weight percent of the reaction mixture in yet other embodiments.

Superatmospheric pressures may be used to supply ample hydrogen to the reaction and to maintain ammonia and methanol in liquid form during the reaction. Hydrogen may be provided to a partial pressure of at least 50 psi (345 kPa) in some embodiments; at least 100 psi (689 kPa) in other embodiments; at least 200 psi (1379 kPa) in other embodiments; and at least 300 psi (2068kPa) in yet other embodiments. Hydrogen partial pressure may be up to 2000 psi (13,790 kPa) in some embodiments; and up to 1200 psi (8274 kPa) in other embodiments (all pressures as measured under reaction conditions). The upper limit on hydrogen pressure is mainly a matter of equipment design; however, little additional benefit is seen by increasing the hydrogen partial pressure above the stated ranges. In other embodiments, hydrogen partial pressures may range from 50 psi (345 kPa) to 250 psi (1724 kPa); and from 100 psi (689 kPa) to 150 psi (1034 kPa) in other embodiments.

Suitable reaction temperatures for some embodiments may be in the range of 40-200 °C. In other embodiments, reaction temperatures may be in the range from 80-160 °C; from 90-140 °C in other embodiments; and from 120-150 °C in yet other embodiments.

Anhydrous ammonia may be used as the ammonia source, although other sources of ammonia may be used as well. Ammonia is typically used in excess of the stoichiometric amount, and in some embodiments at least two moles of ammonia per equivalent of aldehyde groups provided by the starting aldehyde or ketone compound may be used. The amount of ammonia may be as high as 100 moles or more per equivalent of aldehyde or ketone groups provided by the starting aldehyde or ketone compound in some embodiments. In other embodiments, the amount of ammonia may be from 5-60 moles of ammonia per equivalent of aldehyde or ketone groups provided by the starting aldehyde or ketone compound; from 2 to 20 moles of ammonia per equivalent of aldehyde or ketone groups provided by the starting aldehyde or ketone compound in yet other embodiments in other embodiments; and from 7 to 12 moles of ammonia per equivalent of aldehyde or ketone groups provided by the starting aldehyde or ketone compound in yet other embodiments. In selected embodiments, the amount of ammonia may be 4.5 moles of ammonia per equivalent of aldehyde or ketone groups provided by the starting aldehyde or ketone compound.

A hydrogenation catalyst may be present in order to provide a commercially reasonable reaction rate. A wide variety of such catalysts are known, including those described in US 5,055,618 and US 5,041,675. Suitable catalysts are transition metal catalysts, of which the nickel, copper and cobalt catalysts are of particular interest. Nickel catalysts may be selected on the basis of good activity and selectivity, and minimal metal leaching. The catalyst may be an unsupported catalyst such as a Raney nickel or Raney copper catalyst. Supported catalysts may be used as well. Specific examples of suitable catalysts include Raney 2724 (a nickel- and chromium-promoted copper catalyst available from Grace Davison) and especially catalysts Ni-5256 and Ni 0750, both available from Engelhard.

It may be necessary to activate the catalyst prior to the reaction. This is particularly true for non-Raney types of catalysts. Non-Raney catalysts may be activated by heating to a temperature of 100-250 °C in the presence of hydrogen for a period of 0.5 to 5 hours. The catalyst may be slurried in a solvent or diluent during this activation step.

Reaction times may depend on factors such as temperature, hydrogen partial pressure, and type and amount of catalyst. In general, though, a reaction time of from 1.5 to 20 hours is sufficient.

It is believed that in the single-stage process, the product amine compound and the starting aldehyde or ketone compound first react to form relatively low molecular weight intermediates. Because the product amine compound is present in excess (usually in large excess), it is believed that the predominant intermediate that is formed is the reaction product of two molecules of the product amine and one molecule of the starting aldehyde or ketone compound. Most probably, a mixture of intermediates is formed, which represent the reaction products of various ratios of product amine and starting aldehyde or ketone compound.

The single stage process lends itself readily to continuous or semi-continuous operation. During continuous or semi-continuous operation, the starting aldehyde or ketone compound may be added continuously or intermittently to a reaction zone where product amine resides and reductive amination conditions have been established. Other starting materials may be introduced to the reaction zone batch-wise, intermittently, or continuously. Hydrogen may be supplied by pressurizing the reaction zone with hydrogen or a hydrogen-containing mixture of gases and feeding hydrogen on demand. Product may be withdrawn continuously or intermittently as desired, or allowed to accumulate in the reaction mixture.

In the first stage of a two-stage process, the starting aldehyde or ketone compound may be combined with the product amine compound under non-reductive amination conditions to form an intermediate mixture that contains as a primary reaction product, one or more macrocyclic polyimines. In the second stage, the intermediate mixture, or at least a macrocyclic polyimine from the intermediate mixture, is reductively aminated to form the product amine compound.

In the two-stage process, the starting aldehyde or ketone compound may be suitably added to the reaction mixture in an amount from 10 to 35% by weight, based on the combined weight of the starting aldehyde or ketone compound, product amine and methanol that are present at the start of the first reaction step. In some embodiments, the level of aldehyde or ketone compound may vary from 10 to 30% by weight; and from 10 to 25% by weight in other embodiments. A significant advantage of embodiments disclosed herein may be that somewhat high concentrations of reactants as described may be present in the starting solution without significant formation of unwanted high molecular weight polymers or other unwanted reaction by-products. However, greater yield losses may result in a two-stage process when higher concentrations of starting materials are used.

In the two-stage process, the product amine may be suitably added to the first-stage reaction mixture in at least an equimolar amount, based on the amount of starting aldehyde or ketone compound. A small molar excess of the product amine, such as a 5-50% excess may be added in some embodiments, a 10-30% molar excess in other embodiments, as this tends to drive the first step reaction towards the generation of the desired macrocyclic polyimine intermediate material. Generally, an excess of greater than 50 mole percent tends to result in yield losses in the two-stage process.

The product amine compound that is added into the first stage of a two-stage process may be a purified material, or may be a crude product of the reductive amination step, which is partially recycled back to the start of the process. Such a crude amine may include reaction by-products, solvent, ammonia or even small amounts of hydrogen.

The two-stage process is conducted in the presence of methanol. The methanol suitably constitutes from 5 to 90% by weight of the liquid components of the reaction mixture (*i.e*., product amine, intermediates, starting aldehyde or ketone compound and ammonia (if in liquid form)) in some embodiments; and from 10 to 50% by weight of the liquid components of the reaction mixture in yet other embodiments.

The first stage reaction of the starting aldehyde or ketone compound with the product amine in most cases proceeds under mild conditions. At atmospheric pressure and room temperature (∼22 °C), for example, the reactants typically form reaction intermediates within a short period, such as an hour or less, typically 30 minutes or less. The reaction period in some embodiments is at least five minutes. Higher temperatures may be used to accelerate the reaction, but this is generally not necessary. If a higher temperature is used during the first reaction step, it is suitably in the range from 22 to 50 °C in some embodiments, and in the range from 22 to 40 °C in other embodiments. As the reaction of the starting aldehyde or ketone compound and product amine is exothermic, it may be necessary to bring the components together slowly and/or apply cooling to avoid an undesired temperature spike. In the two-stage process, any such temperature spikes may be controlled to below 50 °C in some embodiments, and below 40 °C in other embodiments. Temperatures somewhat lower than room temperature, such as from 0 to 22 °C, may be used if desired, although reaction rates may be slower.

The first reaction stage may be conducted at atmospheric pressure, although higher pressures may be used if desired. Pressures greater than atmospheric may be useful when the reaction mixture contains volatile components (such as ammonia or a solvent such as methanol), in order to prevent those materials from flashing.

Because the hydrogenation reaction may be prevented during the first reaction stage through control of temperature and/or the absence of catalyst, it is possible that ammonia and/or hydrogen may be present during that stage. This may make it possible to use a crude product amine compound (rather than a purified stream) in the first reaction stage.

The formation of intermediates in the first reaction stage of a two-stage process may be detected and followed using analytical methods such as electrospray ionization mass spectroscopy and/or gel permeation chromatography. Alternatively, conditions sufficient to obtain the desired conversion to the intermediates may be established empirically.

The intermediate formed during the first stage of the reaction is believed to consist predominantly (*i.e*., at least 50 % by weight, especially 70-99 % by weight) of macrocyclic polyimine species. A "macrocyclic" polyimine species is a cyclic reaction product of at least two moles of the starting aldehyde or ketone compound with an equal number of moles of the product amine. The macrocyclic polyimine will typically include a mixture of cyclic compounds predominantly having molecular weights of 450 to 1500.

For example, in the case of a cyclohexanedicarboxaldehyde amination, a ∼494 molecular weight species is produced that corresponds to a cyclic reaction product of two moles of the starting cyclohexanedicarboxaldehyde with two moles of the product diamine (A2B2 species, where A represents the starting dicarboxaldehyde and B represents the starting diamine). This macrocyclic intermediate based on the 1,3 isomers may be generically represented by the following structure I:

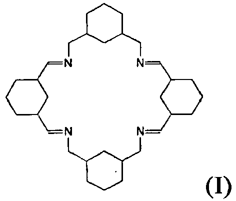

The ∼494 molecular weight product tends to be the most prevalent species. In addition, species corresponding to the cyclic A3B3, A4B4 and A5B5 species are typically present. A ∼1480 molecular weight product is also produced, which corresponds to the cyclic reaction product of six moles of the starting cyclohexanedicarboxaldehyde with six moles of the product diamine (A6B6 species). There are also produced a series of linear species having molecular weights mainly up to 1500, mostly up to 1000. The use of a slight excess of the product diamine tends to favor the production of a minor amount of these linear species. Such linear species may constitute no more than 20% by weight of the reaction products in some embodiments of the two-stage process. In other embodiments, linear species may constitute no more than 10% by weight of the reaction products in some embodiments of the two-stage process; and no more than 5% of the weight of the reaction intermediates in yet other embodiments.

It is believed that such macrocyclic species may also form in some quantities in the one-stage process described before, but that they are rapidly reductively aminated in the one-stage process to form the product amine, and so the macrocyclic species may not accumulate to significant concentrations in the one-stage process.

It is not necessary to recover the intermediate mixture from the solvent or otherwise purify it prior to conducting the amination/hydrogenation reaction in the two-stage process. It is possible to conduct both reaction stages in a single vessel, by conducting the first reaction stage in the presence of the catalyst, and then pressurizing the reaction vessel with ammonia and hydrogen and/or increasing the temperature until the amination/reduction reactions occur. The reactions may be run continuously in a tubular reactor or other suitable apparatus.

The two-stage process may be conducted batch-wise, in a semi-batch operation, or continuously.

A suitable arrangement for a continuous two-stage process includes at least two reactors arranged in series, the first reactor being for the intermediate-forming reaction and the second being for the reductive amination reaction. Starting aldehyde or ketone compound, recycled product amine compound and fresh or recycled methanol as needed is introduced into the entrance of the first reactor. The first reactor is maintained at non-reductive amination conditions described before. The reaction mixture exits the first reactor (after the required residence time) and introduced into the second reactor, together with ammonia and hydrogen feeds. The second reactor contains the catalyst and is operated at reductive amination conditions as described before. Product exiting the second reaction is separated from most or all of the unreacted hydrogen, which may be recycled into the second reactor. The remaining product stream may be separated into an ammonia recycle stream (which is recycled to the second reactor), a byproduct stream (which is sent to disposal or elsewhere), and a product stream. The product stream may be divided between a recycle stream, which is fed back into the first reactor, and final product which may be sent to be purified or to downstream operations (such as phosgenation, when the amine product is to be used as a raw material for polyisocyanate production). Alternatively, the entire product stream may be purified, with a portion of the purified product recycled back to the start of the process.

The aminated and hydrogenated product (from either the one-stage or two-stage embodiments) contains the product amine compound, together with a small amount of reaction by-products. Yields to the desired amine product are typically over 70% in some embodiments, and over 80% in other embodiments, based on the starting aldehyde or ketone compound. Yields are often somewhat higher for the two-stage process than the one-stage process. Yields in a two-stage process are often over 90%. Yields of 93-98% are often achieved in two-stage process. In specific dialdehyde reductive amination reactions, impurities often include one or more bicyclic imine compounds (such as 3-azabicyclo[3.3.1]-2-nonene), and/or bicyclic diamine compounds (such as 2-amino-3-azabicyclo[3.3.1]nonane), both of which are indicative of an incomplete reaction. The bicyclic imine compound may react with additional ammonia to generate the bicyclic diamine, which in turn may be hydrogenated to form the desired product amine compound. Bicyclic amine compounds such as 3-azabicyclo[3.3.1]nonane may also form. The bicyclic amine compounds cannot be easily converted to the desired product. A small amount of other by-products is also produced.

The product amine compound will in most cases exist as a mixture of isomers and, depending on the starting material, may also exist as a mixture of diastereoisomers. Where the product is bis(aminomethyl)cyclohexane, the product may be a mixture of the 1,3- and 1,4-isomers, each of which may exist in both cis- and trans- configurations. The amounts of the 1,3- and 1,4- isomers may be approximately equal. For example, a typically bis(aminomethyl)cyclohexane product mixture may include 45-60% of the 1,3-isomer, and 40-55% of the 1,4-isomer.

The crude product of the reductive amination reaction may include the product amine compound, a small quantity of by-products, unreacted ammonia and hydrogen, and solvent. The product is readily recovered using any convenient methods. Ammonia, hydrogen and solvent may be stripped from the product by venting, applying vacuum, and/or applying an elevated temperature.

The product amine compound may be useful as an intermediate in the synthesis of various downstream products. It may be used as a chain extender or crosslinker for polyurethanes and as an epoxy curing agent. In some embodiments, cycloaliphatic diamines may be used to improve performance and quality attributes of various end products. An application of particular interest may be the manufacture of diisocyanate compounds, which are conveniently formed in the reaction of the amine groups with phosgene. Conditions for conducting such phosgenation reactions are well-known and described, for example, in U. S. Patent Nos. 4,092,343, 4,879,408 and 5,516,935. The diisocyanate compounds are useful in making a wide variety of polyurethane and polyurea polymers.

The following examples are provided to illustrate embodiments disclosed herein, and are not intended to limit the scope thereof. All parts and percentages are by weight unless otherwise indicated.

### Examples

### Example 1

A mixture of 1,3- and 1,4-cyclohexanedicarboxaldehyde (3.08 g, 22 mmol) and a mixture of 1,3- and 1-4-bis(aminomethyl)cyclohexane (4.26 g, 30 mmol) are dissolved in 11 g of methanol. Diglyme (2.38 g) is added as an internal standard for gas chromatographic analysis. The mixture is stirred at room temperature for 30 minutes. During this time, the reactants form an intermediate product mixture containing predominantly macrocyclic polyimine species of about 490 to 1480 molecular weight.

A powdered nickel catalyst (Ni-5256W from Engelhard) (0.75 g) is placed in a 160 mL Parr reactor together with 30 g methanol. The reactor is purged with 100 psi (689 kPa) nitrogen three times, charged with 1000 psi (6895 kPa) hydrogen and heated to 200 °C for two hours to activate the catalyst. The reactor is then cooled and the hydrogen vented off. The intermediate product mixture from above is then transferred into the reactor. Anhydrous ammonia (37.7 g, 2.22 mol) is added with stirring under reduced temperature. The reactor is sealed and pressurized to 300 psi (2068 kPa) with hydrogen. The reactor is then heated to 130 °C with stirring and the hydrogen pressure adjusted to 1000 psi (6895 kPa). These conditions are maintained for five hours, and the reaction contents are recovered. Yield of 1,3- and 1,4-bis(aminomethyl)cyclohexane is 97% by gas chromatography. Isomer ratios are 54.5% of the 1,3-isomer and 45.5% of the 1,4-isomer.

### Example 2

Example 1 is repeated without addition of the diglyme. After the reductive amination is completed, the catalyst is filtered from the reaction mixture and washed twice with methanol (50 g). The wash liquid is combined with the reaction mixture. The methanol is then evaporated off, followed by flash distillation in vacuum at 70-75°C/1 mm Hg to provide 6.61 g of 1,3- and 1,4-bis(aminomethyl)cyclohexane (91% isolated yield).

### Example 3

Example 1 is repeated, except the temperature during the hydrogenation step is only 120°C, the reaction time is 3 hours, and the ratio of ammonia to aldehyde groups provided by the starting mixture of 1,3- and 1,4-cyclohexanedicarboxaldehyde is 25. Yield of 1,3- and 1,4-bis(aminomethyl)cyclohexane is 88%. About 9% 3-azabicyclo[3.3.1]nonane is formed. The presence of the latter species indicates that the amination/reduction reaction has not been completed in the given time at the 120°C temperature and the amount of ammonia that is used.

### Example 4

Example 1 is again repeated, this time reducing the amount of solvent so that the concentration of starting 1,3- and 1,4-cyclohexanedicarboxaldehyde is approximately doubled. The amount of ammonia is decreased so the ratio of moles of ammonia to equivalents of aldehyde groups provided by starting aldehyde is reduced from 50.4 (in Example 1) to about 25. Yield of 1,3- and 1,4-bis(aminomethyl)cyclohexane is 94%. 5% of 3-azabicyclo[3.3.1]nonane is formed. Isomer ratios are 54.6% of the 1,3-isomer and 45.4% of the 1,4-isomer.

### Example 5

Example 1 is repeated again, reducing the amount of methanol so the starting dicarboxaldehyde concentration is approximately triple that used in Example 1. The ammonia/aldehyde group ratio is reduced to about 12.5. Yield of 1,3- and 1,4-bis(aminomethyl)cyclohexane is 93%. Isomer ratios are 55.1% of the 1,3-isomer and 44.9% of the 1,4-isomer.

### Example 6

Example 1 is again repeated, this time reducing the amount of methanol so the starting dicarboxaldehyde concentration is approximately five times that used in Example 1. The ammonia/aldehyde group ratio is reduced to about 6.4. Yield of 1,3-and 1,4-bis(aminomethyl)cyclohexane is 93%. Isomer ratios are 52.9% of the 1,3-isomer and 47.1% of the 1,4isomer.

### Examples 7-9

A powdered Raney nickel catalyst (Ni5256, from Engelhard, 25 g) is ground and added to a 1-gallon autoclave. The reactor is purged with 100 psi (689 kPa) nitrogen three times and 100 g methanol is added. The reactor is then charged with hydrogen, heated to 190°C, and the pressure increased to 1000 psi (6895 kPa) with more hydrogen. The reactor contents are held at these conditions for 2 hours to activate the catalyst. The reactor is then cooled and the hydrogen vented off.

477 grams of a refined bis(aminomethyl)cyclohexane are charged to the reactor followed by 200 grams of methanol. A crude (85 % purity) mixture of 1,3- and 1,4-cyclohexanedicarboxaldehyde) (425 g) is then added slowly with cooling to maintain the temperature of the reaction contents below 40 °C. 100 g of additional methanol are added to rinse feed lines. The solution is then stirred for 30 minutes. 900 g of anhydrous ammonia are added and the reactor is pressurized to 300 psi (2068 kPa) with hydrogen. The reactor is then heated to 130 °C and pressurized to 1000 psi (6895 kPa) with hydrogen. These conditions are maintained for 17 hours, after which the reactor is vented and cooled. The product (Example 7) is collected and analyzed by gas chromatography. Results are as indicated in Table 2 below.

Example 8 is conducted in a similar manner, except that a crude diamine containing about 60 % by weight of the diamine and 20 % by weight of methanol is used instead of the refined material used in Example 7. The diamine is the crude product of a reductive amination similar to Example 7, from which ammonia and hydrogen have been removed. Amination/hydrogenation conditions are maintained for 19.5 hours. Results are as indicated in Table 2 below.

Example 9 is conducted in a manner similar to Example 7, except a crude diamine from a reductive amination similar to Example 7 is used. Hydrogen but not ammonia is removed from the crude diamine. Amination/hydrogenation time is 15 hours. Results are as indicated in Table 2.

Table 1 summarizes the amounts of starting materials used in each of Examples 7-9:

**Table 1**

| | Amount (g) | | |
|---|---|---|---|
| Example No. | 7 | 8 | 9 |
| Added methanol | 400 | 261 | 271 |
| Diamine* | 477 | 708 | 725 |
| Crude dialdehyde (85%) | 425 | 425 | 429 |
| Ammonia | 900 | 800 | 865 |
| Catalyst | 25 | 25 | 25 |
| Hydrogen | 1000 psi (6895 kPa) | 1000 psi (6895 kPa) | 1000 psi (6895 kPa) |

| | | | |
|---|---|---|---|
| *Refined diamine in Example 7; crude diamines in Examples 8 and 9 that contain about 60% by weight of the diamine and 20% by weight of methanol; the crude diamine used in Example 10 also contains ammonia. | | | |

Table 2 summarizes the yield, selectivity and isomer distribution of the products of Examples 7-9. For comparison, the isomer distributions of the starting dialdehyde, refined diamine reactant and crude diamine reactant are provided.

**Table 2**

| | | Isomer Distribution | |
|---|---|---|---|
| Example No. | Selectivity | % 1,3 isomer | % 1,4 isomer |
| 7 | 95 | 54.8 | 45.2 |
| 8 | 90 | 48.6 | 51.4 |
| 9 | 98 | 53.5 | 46.3 |
| Refined Diamine | - | 55.8 | 44.2 |
| Crude Diamine | - | 52.5 | 47.5 |
| Starting dialdehyde | - | 53.3 | 46.7 |

Little change in results is obtained with the variation in diamine feedstock, indicating that a crude diamine reaction product will work well when recycled into the start of the process.

### Comparative Run A

A mixture of 1,3- and 1,4-cyclohexanedicarboxaldehydes (1.017 g; 7.42 mmol), diglyme (0.4033 g, as an internal standard), a Ni catalyst supported on silica/alumina (0.2 g), and methanol (25 ml) are sealed in an 80 ml Parr reactor. Ammonia (6.5 g; 382 mmol) is transferred into the autoclave at ambient temperature. The reactor is heated to 100°C over a 10-15 minute period and kept at that temperature for 30 minutes. Gas chromatography analysis shows complete consumption of the aldehyde. Then 800 psi (5516 kPa) of hydrogen is charged, and the reaction was continued at 100°C at constant hydrogen pressure. After 5 hours, the yields of diamines (1,3- and 1,4-bis(aminomethyl)cyclohexane) and 3-azabicyclo[3.3.1]nonane are 52% and 27%, respectively.

### Example 10

A mixture of 1,3- and 1,4-bis(aminomethyl)cyclohexane isomers is prepared in a semi-batch, one-step process. 10.0 g of cyclohexanedimethyldiamine and 2 g of Engelhard Ni-5256P catalyst are added to a 300 ml autoclave equipped with a stirrer. The reactor is closed and 61.8 g of anhydrous ammonia is added to the reactor while stirring. The reactor is then heated to 120°C to produce a reactor pressure of 1272 psi (8770 kPa). The reactor pressure is increased by an additional 50 psi (345 kPa) by adding hydrogen. A feed burette is charged with a crude mixture of 1,3- and 1,4-cyclohexanedicarboxaldehyde, 86% purity. 53.75 g of the cyclohexanedicarboxaldehyde mixture is pumped into the reactor at a rate of 0.8 ml/min. The total time to pump in the feed is 73 minutes. The feed burette is then flushed with methanol to ensure that all of the cyclohexanedicarboxaldehyde has been fed into the reactor, without introducing a significant quantity of methanol into the reactor. Hydrogen is fed on demand during the cyclohexanedicarboxaldehyde addition, to maintain a constant internal reactor pressure. The reaction is continued after the cyclohexanedicarboxaldehyde addition for a total of about 5 hours. Hydrogen consumption stops after about 120 minutes of reaction time. The reactor is then cooled and vented, and the product is collected. The reactor is rinsed with methanol, and the rinse is collected. 46.2 g of the diamine is produced, for a molar yield of the dialdehyde to the diamine of 87%.

### Comparative Run B

A 300 ml autoclave is charged with 2 g of Engelhard Ni-5256P catalyst and 57.6 g of the crude cyclohexanedicarboxaldehyde described in Example 10. The reactor is pressured with nitrogen and vented. 57 g of anhydrous ammonia are added to the reactor while stirring. The contents are heated to 100°C to produce a reactor pressure of 760 psi (5240 kPa). Hydrogen is added to increase the pressure to 1058 psi (7295 kPa), and hydrogen is thereafter fed on demand to maintain this reactor pressure. The reaction is continued for 7 hours, until hydrogen uptake stops. The total mass of diamine produced is 35 g, which represents a molar yield of the dialdehyde to the diamine of only 69%.

### Example 11

A mixture of 1,3- and 1,4-bis(aminomethyl)cyclohexane isomers is prepared in a semi-batch, one-step process with diamine seed. 9.5 g of cyclohexanedimethyldiamine and Engelhard Ni-5256P catalyst (1.92 g) are added to a 300 mL autoclave equipped with a stirrer. The reactor is closed and 50.4 g of anhydrous ammonia is added to the reactor while stirring. The reactor is then heated to 110°C to produce a reactor pressure of 988 psi. The reactor pressure is increased by additional 115 psi of hydrogen. Methanol (8.7 g) is loaded to the reactor through the dialdehyde feed dip-tube. A feed burette is charged with a crude mixture of 1,3-and 1,4-cyclohexanedicarboxaldehyde, 86% purity. 53.5 g (51 mL) of the cyclohexanedialdehyde mixture is pumped into the reactor at a rate of 0.76 mL/min. The total time to pump the feed is 67 minutes. The dialdehyde feed dip-tube is then flushed with methanol (4.0 g). Hydrogen is fed on demand during the cyclohexanedicarboxaldehyde addition, to maintain a constant internal reactor pressure. The reaction is continued after the cyclohexanedialdehyde addition for a total of about 4 hours. The reactor is then cooled and vented, and the product is collected. 44.7 g of the diamine is produced for a molar yield of the diamine of 95.7% (GC assay). The reaction is repeated for a total of 5 runs with an average yield of 94.5%.

### Comparative Example 1

A mixture of 1,3- and 1,4-bis(aminomethyl)cyclohexane isomers is prepared in a semi-batch, one-step process with diamine seed. 9.5 g of cyclohexanedimethyldiamine and Engelhard Ni-5256P catalyst (1.94 g) are added to a 300 mL autoclave equipped with a stirrer. The reactor is closed and 50.9 g of anhydrous ammonia is added to the reactor while stirring. The reactor is then heated to 100°C to produce a reactor pressure of 856 psi. The reactor pressure is increased by additional 156 psi of hydrogen. Methanol (3.7 g) is loaded to the reactor through the dialdehyde feed dip-tube. A feed burette is charged with a crude mixture of 1,3-and 1,4-cyclohexanedicarboxaldehyde, 86% purity. 54.2 g (51.6 mL) of the cyclohexanedialdehyde mixture is pumped into the reactor at a rate of 0.52 mL/min. The total time to pump the feed is 100 minutes. The dialdehyde feed dip-tube is then flushed with methanol (4.0 g). Hydrogen is fed on demand during the cyclohexanedicarboxaldehyde addition, to maintain a constant internal reactor pressure. The reaction is continued after the cyclohexanedialdehyde addition for a total of about 4 hours. The reactor is then cooled and vented, and the product is collected. 41.6 g of the diamine is produced for a molar yield of the diamine of 88.0%. The reaction is repeated for a total of 5 runs with an average yield of 87.4%.

### Comparative Example 2

A mixture of 1,3- and 1,4-bis(aminomethyl)cyclohexane isomers is prepared in a semi-batch, one-step process with diamine seed. 10.4 g of cyclohexanedimethyldiamine and Engelhard Ni-5256P catalyst (2.03 g) are added to a 300 mL autoclave equipped with a stirrer. The reactor is closed and 55.0 g of anhydrous ammonia is added to the reactor while stirring. The reactor is then heated to 100°C to produce a reactor pressure of 870 psi. The reactor pressure is increased by additional 300 psi of hydrogen. Methanol is not loaded to the reactor prior to the dialdehyde addition. A feed burette is charged with a crude mixture of 1,3- and 1,4-cyclohexanedicarboxaldehyde, 86% purity. 55.65 g (53 mL) of the cyclohexanedialdehyde mixture is pumped into the reactor at a rate of 0.65 mL/min. The total time to pump the feed is 82 minutes. Hydrogen is fed on demand during the cyclohexanedicarboxaldehyde addition, to maintain a constant internal reactor pressure. The reaction is continued after the cyclohexanedialdehyde addition for a total of about 5 hours. The reactor is then cooled and vented, and the product is collected. 39.8g of the diamine is produced for a molar yield of the diamine of 81.9%. The reaction is repeated for a total of 4 runs with an average yield of 81.3%.

### Reference Example 12

A mixture of 1,3- and 1,4-bis(aminomethyl)cyclohexane isomers is prepared in a semi-batch, one-step process with no diamine seed. Engelhard Ni-5256P catalyst (1.81 g) is added to a 300 mL autoclave equipped with a stirrer. No seed product amine is added to the reactor in this Example. The reactor is closed and 47.2 g of anhydrous ammonia is added to the reactor while stirring. The reactor is then heated to 110°C to produce a reactor pressure of 998 psi. The reactor pressure is increased by additional 141 psi of hydrogen. Methanol (7.5 g) is loaded to the reactor through the dialdehyde feed dip-tube. A feed burette is charged with a crude mixture of 1,3-and 1,4-cyclohexanedicarboxaldehyde, 86% purity. 50.2 g (47.8 mL) of the cyclohexanedialdehyde mixture is pumped into the reactor at a rate of 0.71 mL/min. The total time to pump the feed is 68 minutes. The dialdehyde feed dip-tube is then flushed with methanol (3.6 g). Hydrogen is fed on demand during the cyclohexanedicarboxaldehyde addition, to maintain a constant internal reactor pressure. The reaction is continued after the cyclohexanedialdehyde addition for a total of about 4 hours. The reactor is then cooled and vented, and the product is collected. 40.43 g of the diamine is produced for a molar yield of the diamine of 92.3%. The reaction is repeated for a total of 2 runs with an average yield of 91.5%.

Examples 13 through 55 were carried out in manners similar to those described above for Examples 1-12. Reactions were performed as batch, semi-continuous ("semi"), semi-continuous with recycle ("semi-r"), and semi-continuous with added methanol solvent ("semi-m"). Various aldehyde concentrations, ammonia concentrations, reaction temperatures and pressures, and reaction times were used. The results are summarized in Table 3 below. Table 3.

| Example | Run Type | Reaction Temp (°C) | Reaction Pressure (psig) | Ammonia Equivalence | Diamine Loading (eq) | Dialdehyde Feed Rate (mmol/min) | Dialdehyde Feed Rate (meq/min) | Dialdehyde Crude Content (wt.%) | Methanol Content (wt.%) | Reaction Time (hr) | Diamine to BA weight ratio | Yield (%) |
|---|---|---|---|---|---|---|---|---|---|---|---|---|
| Ref13 | Batch | 85 | 1050 | 8.4 | 0.00 | n/a | n/a | 48.5 | 0.0 | 6.0 | -- | 2.0 |
| Ref14 | Batch | 95 | 1100 | 21.1 | 1.14 | n/a | n/a | 20.8 | 0.0 | 7.0 | -- | 79.9 |
| Ref15 | Batch | 95 | 1100 | 22.1 | 1.19 | n/a | n/a | 20.1 | 0.0 | 6.5 | -- | 86.1 |
| Ref16 | Batch | 95 | 1185 | 21.5 | 1.20 | n/a | n/a | 20.3 | 0.0 | 7.0 | -- | 78.8 |
| Ref17 | Semi | 95 | 1200 | 10.6 | 0.21 | 4.75 | 13.9 | 42.9 | 0.0 | 5.0 | -- | 47.9 |
| Ref18 | Semi | 95 | 1200 | 11.0 | 0.21 | 5.08 | 14.9 | 42.1 | 0.0 | 5.0 | -- | 50.2 |
| Ref19 | Semi | 100 | 1400 | 11.1 | 0.21 | 4.50 | 13.2 | 42.1 | 0.0 | 5.0 | -- | 83.2 |
| Ref20 | Semi | 100 | 1100 | 9.5 | 0.21 | 4.16 | 12.2 | 45.2 | 0.0 | 5.0 | -- | 81.9 |
| Ref21 | Semi | 100 | 1400 | 12.4 | 0.21 | 4.88 | 14.3 | 39.6 | 0.0 | 5.0 | -- | 82.5 |
| Ref22 | Semi-r | 100 | 1000 | 9.9 | 0.21 | 5.14 | 14.9 | 43.2 | 0.0 | 5.0 | -- | 78.6 |
| Ref23 | Semi-r | 100 | 1000 | 13.0 | 0.21 | 5.08 | 14.9 | 37.5 | 0.0 | 5.0 | -- | 80.2 |
| Ref24 | Semi-r | 100 | 1050 | 9.3 | 0.21 | 5.08 | 14.9 | 43.7 | 0.0 | 5.0 | 10.6 | 78.1 |
| Ref25 | Semi | 100 | 1200 | 11.9 | 0.22 | 2.97 | 8.7 | 40.6 | 0.0 | 5.0 | 11.4 | 77.7 |
| 26 | Semi-m | 100 | 975 | 10.9 | 0.21 | 4.74 | 13.9 | 40.0 | 5.7 | 5.0 | 14.9 | 86.1 |
| 27 | Semi-m | 100 | 1050 | 9.7 | 0.22 | 4.81 | 14.1 | 42.1 | 6.0 | 4.0 | 17.9 | 88.1 |
| 28 | Semi-m | 90 | 1050 | 9.6 | 0.22 | 4.81 | 14.1 | 42.1 | 6.0 | 4.0 | 16.0 | 86.5 |
| 29 | Semi-m | 90 | 1050 | 8.6 | 0.22 | 4.87 | 14.3 | 44.1 | 6.3 | 4.0 | 18.1 | 84.2 |
| 30 | Semi-m | 90 | 1050 | 9.0 | 0.20 | 7.29 | 21.3 | 43.6 | 6.2 | 4.0 | 13.0 | 83.6 |
| 31 | Semi-m | 90 | 1050 | 9.0 | 0.21 | 3.34 | 10.3 | 43.4 | 6.2 | 4.0 | 15.0 | 84.8 |
| 32 | Semi-m | 90 | 1050 | 9.7 | 0.22 | 4.48 | 13.9 | 42.1 | 6.0 | 5.0 | 14.8 | 86.9 |
| 33 | Semi-m | 90 | 1050 | 8.6 | 0.22 | 5.26 | 16.1 | 44.1 | 6.3 | 5.0 | 12.9 | 79.8 |
| 34 | Semi-m | 100 | 1030 | 8.9 | 0.20 | 6.38 | 16.7 | 43.9 | 6.1 | 4.0 | 12.5 | 88.2 |
| 35 | Semi-m | 100 | 1040 | 9.0 | 0.20 | 3.32 | 10.0 | 43.6 | 6.2 | 4.0 | 17.3 | 88.0 |
| 36 | Semi-m | 100 | 1030 | 8.0 | 0.20 | 4.91 | 13.3 | 45.9 | 6.0 | 4.0 | 16.8 | 86.8 |
| 37 | Semi-m | 100 | 1015 | 9.0 | 0.20 | 6.71 | 20.8 | 41.8 | 10.1 | 4.0 | 24.0 | 87.5 |
| 38 | Semi-m | 110 | 1100 | 9.0 | 0.20 | 4.85 | 14.5 | 43.8 | 6.0 | 4.0 | 28.2 | 90.0 |
| 39 | Semi-m | 100 | 1030 | 4.0 | 0.20 | 4.79 | 10.1 | 57.7 | 6.0 | 4.0 | 7.7 | 71.8 |
| 40 | Semi-m | 110 | 1090 | 9.0 | 0.20 | 4.90 | 14.9 | 41.8 | 9.9 | 4.0 | 36.1 | 95.7 |
| 41 | Semi-m | 100 | 1010 | 9.0 | 0.20 | 3.34 | 10.4 | 43.6 | 6.2 | 4.0 | 17.6 | 88.4 |
| 42 | Semi-m | 120 | 1250 | 9.0 | 0.20 | 5.04 | 14.9 | 43.7 | 6.0 | 4.0 | 32.3 | 91.4 |
| 43 | Semi-m | 110 | 1115 | 9.0 | 0.20 | 4.86 | 14.1 | 45.4 | 2.6 | 4.0 | 18.2 | 89.3 |
| 44 | Semi-m | 110 | 1100 | 9.0 | 0.20 | 4.86 | 16.1 | 41.8 | 10.0 | 4.0 | 25.2 | 93.9 |
| 45 | Semi-m | 110 | 1120 | 9.0 | 0.23 | 4.77 | 13.5 | 41.9 | 9.8 | 4.0 | 28.0 | 95.7 |
| 46 | Semi-m | 110 | 1125 | 9.0 | 0.24 | 4.74 | 14.9 | 41.0 | 9.8 | 4.0 | 29.1 | 92.8 |
| 47 | Semi-m | 110 | 1130 | 9.0 | 0.23 | 4.74 | 14.3 | 41.3 | 9.9 | 4.0 | 40.4 | 48.8 |
| 48 | Semi-m | 110 | 1120 | 8.2 | 0.19 | 5.15 | 15.2 | 44.4 | 9.6 | 6.5 | 16.7 | 82.5 |
| 49 | Semi-m | 110 | 1130 | 9.0 | 0.20 | 4.00 | 117.0 | 41.4 | 11.1 | 4.75 | 16.9 | 91.6 |
| 50 | Semi-m | 110 | 1130 | 9.0 | 0.20 | 4.80 | 13.7 | 41.9 | 9.9 | 4.0 | 41.9 | 94.4 |
| 51 | Semi-m | 110 | 1130 | 9.0 | 0.20 | 4.80 | 14.9 | 41.5 | 10.0 | 4.0 | 41.4 | 93.0 |
| 52 | Semi-m | 110 | 1150 | 9.0 | 0.20 | 4.81 | 14.9 | 43.6 | 6.6 | 6.5 | -- | |
| 53 | Semi-m | 110 | 1125 | 9.0 | 0.00 | 4.83 | 15.4 | 45.6 | 10.0 | 4.0 | 30.6 | 90.6 |
| 54 | Semi-m | 110 | 1140 | 9.0 | 0.00 | 4.60 | 14.9 | 45.5 | 10.0 | 4.0 | 31.9 | 92.3 |
| 55 | Semi-m | 120 | 1250 | 9.0 | 0.00 | 4.81 | 21.7 | 45.6 | 10.0 | 4.0 | 21.6 | 90.9 |

| | | | | | | | | | | | | |
|---|---|---|---|---|---|---|---|---|---|---|---|---|
| [Ref = reference example, not within the scope of the claims] | | | | | | | | | | | | |

Advantageously, embodiments disclosed herein may provide for diamines to be produced more efficiently and with less waste. As described above, it has been shown that under certain reaction conditions the yield of the reductive amination reaction of cyclohexanedicarboxaldehydes may be increased. In some embodiments described herein, diamine yield may be in excess of 90 percent with or without use of diamine product seed present during the reductive amination reaction. In other embodiments, methanol may be used to increase diamine yield.

## Claims

1. A method for reductively aminating a starting aldehyde or ketone compound having at least two aldehyde or ketone groups per molecule to form a product amine compound, comprising (a) mixing the starting aldehyde or ketone compound with a quantity of the product amine compound and methanol to form a liquid mixture, and (b) subjecting the liquid mixture to reductive amination conditions in the presence of ammonia and hydrogen to produce additional product amine compound, wherein during steps a) and b) the molar ratio of product amine compound to starting aldehyde or ketone compound in the mixture is 1:1 or greater.

2. The method of claim 1, wherein steps a) and b) are conducted simultaneously.

3. The method of claim 1, wherein steps a) and b) are conducted continuously or semicontinuously.

4. The method of claim 3, wherein the starting aldehyde or ketone compound is a cycloaliphatic aldehyde or ketone compound in which the carbonyl carbon atoms of the aldehyde or ketone groups are attached to an aliphatic ring structure.

5. The method of claim 4, wherein the starting aldehyde or ketone compound is a dialdehyde compound.

6. The method of claim 5 wherein the dialdehyde compound is a 1,3-cyclohexanedicarboxaldehyde, 1,4-cyclohexanedicarboxaldehyde, or a mixture thereof and the product amine is 1,3-bis(aminomethyl)cyclohexane, 1,4-bis(aminomethyl)cyclohexane or a mixture thereof.

7. The method of claim 2, wherein step a) is conducted under non-reductive amination conditions.

8. The method of claim 7, wherein the concentration of the starting aldehyde or ketone in the liquid mixture is from 10 to 30% by weight, based on the combined weights of the starting aldehyde or ketone, the product amine compound used in step a), and the methanol.

9. The method of claim 8, wherein in step a), at least 50% by weight of the reaction intermediates formed are macrocyclic polyimines having molecular weights of 450 to 1500.

10. The method of claim 9, wherein in step a), a 10 to 30% molar excess of the product amine compound is mixed with the starting aldehyde or ketone compound, based on the amount of the starting aldehyde or ketone compound, to form the liquid mixture.

11. The method of claim 7, wherein the starting aldehyde or ketone compound is a cycloaliphatic aldehyde or ketone compound in which the carbonyl carbon atoms of the aldehyde or ketone groups are attached to an aliphatic ring structure.

12. The method of claim 11, wherein the starting aldehyde or ketone compound is a dialdehyde compound.

13. The method of claim 12, wherein the dialdehyde compound is a 1,3-cyclohexanedicarboxaldehyde, 1,4-cyclohexanedicarboxaldehyde, or a mixture thereof and the product amine is 1,3-bis(aminomethyl)cyclohexane, 1,4-bis(aminomethyl)cyclohexane or a mixture thereof.

14. The method of claim 2 for reductively aminating a starting aldehyde or ketone compound having at least two aldehyde or ketone groups per molecule to form a product amine compound, comprising
a) mixing the product amine compound and starting aldehyde or ketone compound at a molar ratio of at least 1:1 and methanol to form the liquid mixture, and maintaining the liquid mixture under non-reductive amination conditions sufficient to form an intermediate mixture containing reaction intermediates formed from the product amine compound and the starting aldehyde or ketone compound, which reaction intermediates consist predominantly of one or more macrocyclic polyimine compounds; and
b) thereafter subjecting at least one of the macrocyclic polyimine compounds to reductive amination conditions in the presence of ammonia and hydrogen to convert the macrocyclic polyimine compound to the product amine compound.

15. The method of claim 14,
wherein the concentration of the starting aldehyde or ketone in the liquid
mixture is from 10 to 30% by weight, based on the combined weights of the starting aldehyde or ketone compound, the product amine compound used in step a), and the methanol;
wherein in step a), at least 50% by weight of the reaction intermediates
formed are macrocyclic polyimines having molecular weights of 450 to 1500;
wherein in step a), a 10 to 30% molar excess of the product amine compound
is mixed with the starting aldehyde or ketone compound, based on the amount of aldehyde or ketone compound, to form the liquid mixture;
wherein step a) is conducted at a temperature of 0-50°C for a period of 5
minutes to one hour;
wherein the starting aldehyde compound is 1,3-cyclohexanedicarboxaldehyde,
1,4-cyclohexanedicarboxaldehyde, or a mixture thereof and the product amine is 1,3-bis(aminomethyl)cyclohexane, 1,4-bis(aminomethyl)cyclohexane or a mixture thereof.

## Patentansprüche

1. Ein Verfahren zum reduktiven Aminieren einer Aldehyd- oder
Ketonausgangsverbindung, die mindestens zwei Aldehyd- oder Ketongruppen pro Molekül aufweist, um eine Aminproduktverbindung zu bilden, beinhaltend (a) Mischen der Aldehyd- oder Ketonausgangsverbindung mit einer Quantität der Aminproduktverbindung und Methanol, um eine flüssige Mischung zu bilden, und (b) Aussetzen der flüssigen Mischung gegenüber reduktiven Aminierungsbedingungen in Gegenwart von Ammoniak und Wasserstoff, um zusätzliche Aminproduktverbindung zu produzieren, wobei das molare Verhältnis der Aminproduktverbindung zur Aldehyd- oder Ketonausgangsverbindung in der Mischung während der Schritte a) und b) 1 : 1 oder höher ist.

2. Verfahren gemäß Anspruch 1, wobei die Schritte a) und b) gleichzeitig ausgeführt werden.

3. Verfahren gemäß Anspruch 1, wobei die Schritte a) und b) kontinuierlich oder halbkontinuierlich ausgeführt werden.

4. Verfahren gemäß Anspruch 3, wobei die Aldehyd- oder Ketonausgangsverbindung eine cycloaliphatische Aldehyd- oder Ketonverbindung ist, bei der die Carbonylkohlenstoffatome der Aldehyd- oder Ketongruppen an einer aliphatischen Ringstruktur hängen.

5. Verfahren gemäß Anspruch 4, wobei die Aldehyd- oder Ketonausgangsverbindung eine Dialdehydverbindung ist.

6. Verfahren gemäß Anspruch 5, wobei die Dialdehydverbindung ein 1,3-Cyclohexandicarboxaldehyd, 1,4-Cyclohexandicarboxaldehyd oder eine Mischung davon ist und das Aminprodukt 1,3-Bis(Aminomethyl)cyclohexan, 1,4-Bis(Aminomethyl)cyclohexan oder eine Mischung davon ist.

7. Verfahren gemäß Anspruch 2, wobei Schritt a) unter nichtreduktiven Aminierungsbedingungen ausgeführt wird.

8. Verfahren gemäß Anspruch 7, wobei die Konzentration des Ausgangsaldehyds oder -ketons in der flüssigen Mischung von 10 bis 30 Gew.-%, bezogen auf die kombinierten Gewichte des Ausgangsaldehyds oder -ketons, der in Schritt a) verwendeten Aminproduktverbindung und des Methanols, beträgt.

9. Verfahren gemäß Anspruch 8, wobei in Schritt a) mindestens 50 Gew.-% der gebildeten Reaktionszwischenprodukte makrocyclische Polyimine mit Molekulargewichten von 450 bis 1500 sind.

10. Verfahren gemäß Anspruch 9, wobei in Schritt a) ein 10- bis 30%iger molarer Überschuss der Aminproduktverbindung mit der Aldehyd- oder Ketonausgangsverbindung, bezogen auf die Menge der Aldehyd- oder Ketonausgangsverbindung, gemischt wird, um die flüssige Mischung zu bilden.

11. Verfahren gemäß Anspruch 7, wobei die Aldehyd- oder Ketonausgangsverbindung eine cycloaliphatische Aldehyd- oder Ketonverbindung ist, in der die Carbonylkohlenstoffatome der Aldehyd- oder Ketongruppen an einer aliphatischen Ringstruktur hängen.

12. Verfahren gemäß Anspruch 11, wobei die Aldehyd- oder Ketonausgangsverbindung eine Dialdehydverbindung ist.

13. Verfahren gemäß Anspruch 12, wobei die Dialdehydverbindung ein 1,3-Cyclohexandicarboxaldehyd, 1,4-Cyclohexandicarboxaldehyd oder eine Mischung davon ist und das Aminprodukt 1,3-Bis(Aminomethyl)cyclohexan, 1,4-Bis(Aminomethyl)cyclohexan oder eine Mischung davon ist.

14. Verfahren gemäß Anspruch 2 zum reduktiven Aminieren einer Aldehyd- oder Ketonausgangsverbindung, die mindestens zwei Aldehyd- oder Ketongruppen pro Molekül aufweist, um eine Aminproduktverbindung zu bilden, beinhaltend
a) Mischen von der Aminproduktverbindung und der Aldehyd- oder Ketonausgangsverbindung in einem molaren Verhältnis von mindestens 1 : 1 und Methanol, um die flüssige Mischung zu bilden, und Halten der flüssigen Mischung unter nichtreduktiven Aminierungsbedingungen, die ausreichen, um eine Zwischenproduktmischung zu bilden, welche Reaktionszwischenprodukte enthält, die aus der Aminproduktverbindung und der Aldehyd- oder Ketonausgangsverbindung gebildet sind, wobei die Reaktionszwischenprodukte hauptsächlich aus einer oder mehr makrocyclischen Polyiminverbindungen bestehen; und
b) danach Aussetzen mindestens einer der makrocyclischen Polyiminverbindungen gegenüber reduktiven Aminierungsbedingungen in Gegenwart von Ammoniak und Wasserstoff, um die makrocyclische Polyiminverbindung in die Aminproduktverbindung umzuwandeln.

15. Verfahren gemäß Anspruch 14,
wobei die Konzentration des Ausgangsaldehyds oder -ketons in der flüssigen Mischung von 10 bis 30 Gew.-%, bezogen auf die kombinierten Gewichte der Aldehyd- oder Ketonausgangsverbindung, der in Schritt a) verwendeten Aminproduktverbindung und des Methanols, beträgt;
wobei in Schritt a) mindestens 50 Gew.-% der gebildeten Reaktionszwischenprodukte makrocyclische Polyimine mit Molekulargewichten von 450 bis 1500 sind;
wobei in Schritt a) ein 10- bis 30%iger molarer Überschuss der Aminproduktverbindung, bezogen auf die Menge der Aldehyd- oder Ketonverbindung, mit der Aldehyd- oder Ketonausgangsverbindung gemischt wird, um die flüssige Mischung zu bilden; wobei Schritt a) über einen Zeitraum von 5 Minuten bis einer Stunde bei einer Temperatur von 0-50 °C ausgeführt wird;
wobei die Aldehydausgangsverbindung 1,3-Cyclohexandicarboxaldehyd, 1,4-Cyclohexandicarboxaldehyd oder eine Mischung davon ist und das Aminprodukt 1,3-Bis(Aminomethyl)cyclohexan, 1,4-Bis(Aminomethyl)cyclohexan oder eine Mischung davon ist.

## Revendications

1. Une méthode pour aminer de façon réductrice un composé aldéhyde ou cétone de départ ayant au moins deux groupes aldéhyde ou cétone par molécule pour former un composé amine produit, comprenant (a) le mélange du composé aldéhyde ou cétone de départ avec une quantité du composé amine produit et du méthanol pour former un mélange liquide, et (b) la soumission du mélange liquide à des conditions d'amination réductrices en présence d'ammoniac et d'hydrogène pour produire du composé amine produit supplémentaire, dans laquelle au cours des étapes a) et b) le rapport molaire du composé amine produit au composé aldéhyde ou cétone de départ dans le mélange est de 1/1 ou plus.

2. La méthode de la revendication 1, dans laquelle les étapes a) et b) sont menées simultanément.

3. La méthode de la revendication 1, dans laquelle les étapes a) et b) sont menées en continu ou en semi-continu.

4. La méthode de la revendication 3, dans laquelle le composé aldéhyde ou cétone de départ est un composé aldéhyde ou cétone cycloaliphatique dans lequel les atomes de carbone carbonyle des groupes aldéhyde ou cétone sont attachés à une structure cyclique aliphatique.

5. La méthode de la revendication 4, dans laquelle le composé aldéhyde ou cétone de départ est un composé dialdéhyde.

6. La méthode de la revendication 5 dans laquelle le composé dialdéhyde est un 1,3-cyclohexane dicarboxaldéhyde, un 1,4-cyclohexane dicarboxaldéhyde, ou un mélange de ceux-ci et l'amine produit est le 1,3-bis(aminométhyl)cyclohexane, le 1,4-bis(aminométhyl)cyclohexane ou un mélange de ceux-ci.

7. La méthode de la revendication 2, dans laquelle l'étape a) est menée sous des conditions d'amination non réductrices.

8. La méthode de la revendication 7, dans laquelle la concentration de l'aldéhyde ou de la cétone de départ dans le mélange liquide va de 10 à 30 % en poids, rapporté aux poids combinés de l'aldéhyde ou de la cétone de départ, du composé amine produit utilisé dans l'étape a), et du méthanol.

9. La méthode de la revendication 8, dans laquelle dans l'étape a), au moins 50 % en poids des intermédiaires de réaction formés sont des polyimines macrocycliques ayant des masses moléculaires de 450 à 1 500.

10. La méthode de la revendication 9, dans laquelle dans l'étape a), un excès molaire de 10 à 30 % du composé amine produit est mélangé avec le composé aldéhyde ou cétone de départ, rapporté à la quantité du composé aldéhyde ou cétone de départ, pour former le mélange liquide.

11. La méthode de la revendication 7, dans laquelle le composé aldéhyde ou cétone de départ est un composé aldéhyde ou cétone cycloaliphatique dans lequel les atomes de carbone carbonyle des groupes aldéhyde ou cétone sont attachés à une structure cyclique aliphatique.

12. La méthode de la revendication 11, dans laquelle le composé aldéhyde ou cétone de départ est un composé dialdéhyde.

13. La méthode de la revendication 12, dans laquelle le composé dialdéhyde est un 1,3-cyclohexane dicarboxaldéhyde, un 1,4-cyclohexane dicarboxaldéhyde, ou un mélange de ceux-ci et l'amine produit est le 1,3-bis(aminométhyl)cyclohexane, le 1,4-bis(aminométhyl)cyclohexane ou un mélange de ceux-ci.

14. La méthode de la revendication 2 pour aminer de façon réductrice un composé aldéhyde ou cétone de départ ayant au moins deux groupes aldéhyde ou cétone par molécule pour former un composé amine produit, comprenant
a) le mélange du composé amine produit et du composé aldéhyde ou cétone de départ à un rapport molaire d'au moins 1/1 et de méthanol pour former le mélange liquide, et le maintien du mélange liquide sous des conditions d'amination non réductrices suffisantes pour former un mélange intermédiaire contenant des intermédiaires de réaction formés à partir du composé amine produit et du composé aldéhyde ou cétone de départ, lesquels intermédiaires de réaction consistent de façon prédominante en un ou plusieurs composés polyimine macrocyclique ; et
b) la soumission ensuite d'au moins un des composés polyimine macrocyclique à des conditions d'amination réductrices en présence d'ammoniac et d'hydrogène pour convertir le composé polyimine macrocyclique en composé amine produit.

15. La méthode de la revendication 14,
dans laquelle la concentration de l'aldéhyde ou de la cétone de départ dans le mélange liquide va de 10 à 30 % en poids, rapporté aux poids combinés du composé aldéhyde ou cétone de départ, du composé amine produit utilisé dans l'étape a), et du méthanol ; dans laquelle dans l'étape a), au moins 50 % en poids des intermédiaires de réaction formés sont des polyimines macrocycliques ayant des masses moléculaires de 450 à 1 500 ;
dans laquelle dans l'étape a), un excès molaire de 10 à 30 % du composé amine produit est mélangé avec le composé aldéhyde ou cétone de départ, rapporté à la quantité du composé aldéhyde ou cétone, pour former le mélange liquide ;
dans laquelle l'étape a) est menée à une température de 0 à 50 °C pendant une période allant de 5 minutes à une heure ;
dans laquelle le composé aldéhyde de départ est un 1,3-cyclohexane dicarboxaldéhyde, un 1,4-cyclohexane dicarboxaldéhyde, ou un mélange de ceux-ci et l'amine produit est le 1,3-bis(aminométhyl)cyclohexane, le 1,4-bis(aminométhyl)cyclohexane ou un mélange de ceux-ci.
